# EUROPEAN PATENT APPLICATION

(11) **EP 2 859 945 A1**
(43) Date of publication of application: **15.04.2015**
(21) Application number: 13382403.7
(22) Date of filing: 11.10.2013
(51) Int. Cl.: B01J 37/08, B01J 21/06, B01J 21/08, B01J 37/02, C07D 301/19

(54) **Process for the preparation of a heterogeneous epoxidation catalyst**

(71) Applicant: Repsol, S.A., 28045 Madrid (ES)
(72) Inventor: VEGA BERMEJO, Luis, 28935 MÓSTOLES (ES); MASA LORENZO, María de la O, 28935 MÓSTOLES (ES); CARAZO ANGULO, José Antonio, 28935 MÓSTOLES (ES); MARTÍNEZ SILVESTRE, Raquel, 28934 MÓSTOLES (ES)
(74) Representative: ZBM Patents - Zea, Barlocci & Markvardsen

(57) **Abstract**

A process for preparing a heterogeneous titanium-containing silicon oxide catalyst comprising the steps of impregnating a siliceous carrier with a titanium source, calcinating in a very precise conditions of temperature and time and optionally hydrolyzing and silylating the catalyst; as well as a process for the preparation of an alkylene oxide comprising reacting an olefin with an organic hydroperoxide in the presence of the heterogeneous titaniumbased catalyst prepared in accordance with the process of the invention.

## Description

The present invention relates to a process for the preparation of a titanium-containing heterogeneous epoxidation catalyst, and its use in an industrial scale epoxidation of olefins.

### BACKGROUND ART

The basic technology for the epoxidation of olefins by heterogeneous catalysis is known from more than 40 years. Generally, the process for the epoxidation of olefins to obtain an oxirane product, involves the reaction of the olefin with an organic hydroperoxide in the presence of various catalyst materials.

For instance, a commonly known method for manufacturing propylene oxide is the co-production of propylene oxide and styrene starting from ethylbenzene. In general such process involves the steps of (i) reacting ethylbenzene (EB) with oxygen or air to form ethylbenzene hydroperoxide (EBHP), (ii)reacting the ethylbenzene hydroperoxide (EBHP) thus obtained with propene in the presence of an epoxidation catalyst to yield propylene oxide (PO) and 1-phenyl ethanol (methyl phenyl carbinol, MPC), and (iii) dehydrating the 1-phenyl ethanol (MPC) into styrene (SM) using a suitable dehydration catalyst.

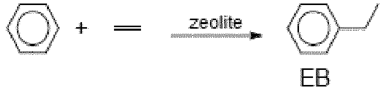

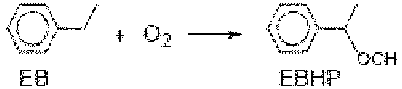

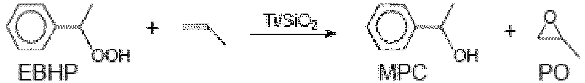

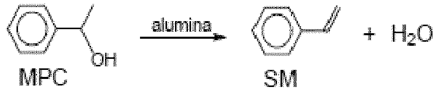

Epoxidation catalysts can be homogenous or heterogeneous. Manufacturing methods for heterogeneous epoxidation catalyst, using a silica support are well known in the art. These methods are complex and comprise a number of steps: different pretreatments of the siliceous-based support, different impregnation process and different finalizations steps.

It is known that previously to the impregnation of the titanium compound on the siliceous-based support, the siliceous can be treated previously with acid. For example, in US5932751 it is described a process wherein the siliceous-based support has been pretreated by acid washing prior to the deposition of the titanium component thereon, followed by a washing step with deionized water and opcionally with alcohol to remove any residual traces of acid treating material (halides, sulfates...) that could interfere with the anchoring of the titanium and especially with the catalyst reaction. The undesirable effects derived from the presence of such acid materials is known in the art, for example in "Halide Effects in Transition Metal Catalysis" Keith Fagnou and Mark Lautens. Angewandte. Chemie. Int. Ed. 2002, 41, 26 - 47, wherein it is stated that among the most common ligands found on transition metal catalysts are halide ions. It is mentioned that of the commercially available catalysts or pre-catalysts, most are halo metal complexes; and in recent years, manipulation of this metal-halide functionality has revealed that this can be used as a highly valuable method of tuning the reactivity of the complex. Variation of the halide ligand will usually not alter the nature of the system to the extent that it becomes unreactive but will impart sufficiently large changes that differences in reactivity or selectivity occur. Additionally, in "Mecanism in Homogeneus and heterogeneus epoxidation catalyst" S.Ted Oyama. Ed Elsevier, Pag 251-252, it is mentioned that the principal promoter used in EO (Ethylene Oxide) production is the Cl atom. It is introduced to de Ag by adding vinyl chloride or dichloroethane in ppm quantities to the feed.

It has been reported in the prior art that using this acid treatment is observed an improvement in activity (% conversion) of the catalyst in the epoxidation reaction, nevertheless it also results in a decrease in selectivity.

Titanium compound can be supported on the siliceous-based support by a gas phase supporting method or a liquid phase supporting method. In the gas phase supporting, titanium salts and titanates of organic acids and inorganic acids, which are able to vaporize at supporting temperature, are applied onto the siliceous-based support. In the liquid phase supporting method the siliceous-based support is put in contact with salts and titanates of organic acids and inorganic acids, which can be solved in a suitable solvent.

Supported titanium catalysts require one or more calcination stages during its manufacture. This step is expensive, intensive in energy consumption and in nitrogen or other inert gas. The duration of this stage is given not only by the calcination time, but also by very slow heating and cooling ramps, reported in the order of 50 °C/h. Using ordinary calcining temperatures, it represents to double or triple the operating time. These ramps are made traditionally to avoid damaging the physical properties of the porous structure of the silica support by thermal shock breakage.

Thus, in "Fundamentos de ciencia de los materiales" Tomo I. Vicente Amigó Borrás; Carlos Ferrer Giménez; María Dolores Salvador Moya; Francisco Segovia López; Alfonso Cristóbal Cárcel González; Universidad Politécnica de Valencia Departamento de Ingeniería Mecánica y de Materiales Valencia : Universidad Politécnica de Valencia 1994, Chapter 11 Características térmicas de los materiales, apartado 4.5: Efecto de los gradientes de temperatura: roturas por choque térmico. (page 405), it is mentioned that chipping or breakage due to thermal shock is a phenomenon that is associated with sudden temperature changes, especially in fragile materials such as glass and ceramics.

Considering these heating rates, calcination as described, must be performed in discontinuous batches in tray dryers or similar, with the implicit drawbacks of batch manufacturing and the difficulty of maintaining an inert atmosphere.

Generally, the calcining temperature is between 400-900 °C, with a heating ramp of 50 °C/h and usual calcining time is from 4-18 hours. For example, in EP345856 it is described the use of a calcining step wherein the impregnated silica is heated in a nitrogen atmosphere to 600 °C at a rate of 50 °C/h and calcined for 4 hours at 600 °C; the calcined catalyst is cooled at 300 °C and steam is added to the circulating nitrogen which flow at a rate of 20 l per hour over the catalyst, steam treatment is carried out for 2 hours; the reactor is then cooled to 200 °C in a stream of dry nitrogen. In EP1292391, the impregnated silica is heated in a nitrogen atmosphere to 600 °C at a rate of 50 °C/h and calcinated for 6 hours at 600 °C; the calcined catalyst is cooled to 325 °C, while the nitrogen flow is increased to 10 NL/h; then steam is added to the nitrogen circulating over the catalyst at a rate of 4 g/h for 2 hours at 325 °C; the reactor is then cooled to 200 °C.

There exists the neccesity of an improved process for the preparation of a titanium based catalyst which is industrially scalable, less time and energy consuming, and which results in a catalyst having high activity and selectivity in the epoxidation of olefins.

### SUMMARY OF THE INVENTION

The present invention provides a preparation process of a titanium containing solid catalyst used for producing an oxirane compound by reacting an olefin type compound with an organic hydroperoxide, the catalyst having high activity and selectivity. The process being cost effective and industrially scalable.

Accordingly, the present invention provides a process for the preparation of a heterogeneous titanium based catalyst obtained by a process characterized in that once the siliceous-based support is impregnated with the titanium-containing agent, an accelerated heating rate is applied thereon; and afterwards the temperature of calcination reached after each ramp is maintained for at least 2 hours in order to calcinate the impregnated carrier.

In particular, in accordance with a first aspect of the present invention, it is provided a process for the preparation of a heterogeneous titanium based catalyst comprising the steps of:
i) contacting the siliceous-based carrier with a titanium-containing agent to obtain a titanium impregnated carrier;
ii) raising the temperature up to 450-1000 °C, applying at least one temperature ramp of between 100-1000 °C/h, and maintaining the temperature reached after each ramp from 2 to 24 hours in order to calcinate the impregnated carrier of step i).

It is also an aspect of the present invention a process for the preparation of an alkylene oxide by reacting an olefin with an organic hydroperoxide in the presence of a heterogeneous catalyst, wherein the catalyst has been obtained by a process comprising the steps of:
i) contacting a siliceous-based carrier with a titanium-containing agent to obtain a titanium impregnated carrier;
ii) raising the temperature up to 450-1000 °C, applying at least one temperature ramp of between 100-1000 °C/h, and maintaining the temperature reached after each ramp from 2 to 24 hours in order to calcinate the impregnated carrier of step i).

### DETAILED DESCRIPTION OF THE INVENTION

Siliceous-based supports suitable as carriers for the preparation process according to the present invention are any previously known materials generally used in epoxidation process. Preferably, the siliceous-based carrier is an amorphous silica. In the context of the present invention, the term amorphous silica" is used here for the pure forms of SiO₂ such as colloidal silica, precipitated silica, silica gel, pyrogenic silica, silica fume, quartz glass, fused silica. Particularly preferred are amorphous precipitated silicas and amorphous pyrogenic silicas.

Synthetic amorphous silicas can be obtained according to any of the known methods in the art.

In accordance with an embodiment of the present invention, the specific surface area of the siliceous-based support is between from 150-300m²/g, preferably from 120-250 m²/g, ASTM D3663 - 03(2008); a Pore Size Distribution (as determined by Nitrogen Desorption Isotherms, method ASTM: 4641-12) in the range of from 0.7-1.0 cm³/g, preferably from 0.78-0.90 cm³/g; and average pore diameter as determined by Nitrogen Isotherm, using de BJH Method (Barrett, E.P.; L.G. Joyner, P.P. Halenda (1951). "The Determination of Pore Volume and Area Distributions in Porous Substances. I. Computations form Nitrogen Isotherms". J. Am. Chem. Soc. 73), in the range of from 20-300 amgstroms, preferably from 35-165 amgstroms.

According to an embodiment of the present invention, the process for the preparation of the catalyst further comprises, in addition to steps i) to ii) mentioned hereinbefore and previously to step i), an acid pretreatment of the siliceous-based support.

The acid pretreatment of the siliceous-based support can be carried out using known methods in the art. Thus, in accordance with an embodiment of the present invention, the acid pretreatment is carried out with an aqueous solution of a strong acid; suitable acids are sulfuric acid, hydrochloric acid, phosphoric acid, nitric acid, acetic acid, and the like. Preferably, an aqueous solution of hydrochloric acid is used. Therefore, in accordance with a particular embodiment of the present invention the acid treatment is carried out by immersion of the siliceous compound carrier in the acidic solution, preferably an aqueous solution of hydrochloric acid, preferably at a temperature ranged from 20 to 90 °C; for a period of from 1 - 24 h. More preferably, at a temperature ranged from 60 - 70 °C for a period from 2 - 6 hours, more preferably from 3 - 4 hours.

In one embodiment of the invention, the catalyst preparation method is characterized by submerging the siliceous-based support in hydrochloric acid, wherein the concentration is in the range from 0,1 moles/liter to 12 moles/liter; preferably from 1 to 10 moles/liter.

At the completion of this acid treatment, the treated siliceous-based support is separated from the aqueous acid by any known method, e.g. filtration.

In accordance with a preferred embodiment of the preparation process of the present invention, between the acid pretreatment step and the impregnation with the Ti-containing agent, no further treatment of the carrier, such as aqueous or alcoholic washing, takes place, i.e. the impregnation step is carried out directly after the acid treatment.

Surprisingly, it has been demonstrated, contrary to what it is mentioned in the prior art, that it is not neccesary to carry out an aqueous or alcoholic washing of the acid treated support before the impregnation step (c.f. data shown in the examples) while mantaining an improvement in activity, and wherein lost in selectivity is reduced

The absence of an aqueous or alcoholic washing step after the acidic pretreatment, allows to reduce the cost of the the preparation process, since the washing step generally suppose a consumption of around 20 g water/g support. Thus, resulting in a more envirommentally friendly and cost efficient process.

After the acid treatment of the siliceous-based carrier, the pretreated carrier can be dried at a moderate temperature. After that optional drying step, the siliceous-based carrier is heated at a temperature from 150-300 °C during 1-4 hours under inert atmosphere. If present, the first drying step is carried out at a temperature ranging from 100 to 200 °C, preferably from 105 to 150 °C, being particularly preferred from 110 to 130 °C. The second drying step is carried out at a temperature from 100 - 300 °C, preferably from 150 - 250 °C, particularly preferably from 200 - 220 °C; during 1-4 hours, preferably during 2-3 hours, under nitrogen atmosphere.

It is highly desirable to dry the inorganic siliceous solid prior to impregnation because titanium tetrachloride reacts with water forming white fumes of hydrochloric acid. A flowing stream of a dry gas such as nitrogen must be applied to dry the carrier, while fumes are formed upon contact with atmospheric humidity.

Thus, in accordance with an embodiment of the present invention, a drying step is carried out directly on the acid treated siliceous-based carrier, with no aqueous or alcoholic washing steps between acid treatment and drying steps.

Once it is dried, the acid treated catalyst is impregnated with a titanium-containing agent.

As mentioned above, the acid treatment of the siliceous-based carrier is an optional step. Obviously, if no acid treatment is performed, the drying step can also be partially avoided, always it is necessary to dry before using TiCl₄, therefore at least the second drying step under inert atmosphere is advisable.

The impregnation step is not limited by choice of a particular titanium source, nor by any particular impregnation method. Thus, as preferred titanium source, it can be used titanium alkoxides and titanium halides, being titanium tetrachloride particularly preferred. Particularly preferred methods of impregnation include liquid-phase and vapor-phase impregation methods, which are well known in the art.

In the liquid phase supporting method impregnation may by performed by dissolving a titanium halide or titanium alkoxide (such as TiCl₄ or Ti(OiPr)₄)) in an organic solvent and then applying the solution to the pretreated spent catalyst. Suitable solvents for this purpose are those hydrocarbons that are liquid at ambient temperatures, and are capable of solubilizing the titanium compound. Examples of suitable hydrocarbon solvents include n-hexane, n-heptane, cyclopentane, methyl pentanes, methyl cyclohexane, dimethyl hexanes, toluene, xylenes, methylene chloride, chloroform, dichloroethanes, chlorobenzene, benzyl chloride, and the like.

In accordance with a particular realisation of the liquid-phase impregnation method, the titanium compound for impregnation is Ti(OiPr)₄) and the solvent is iPrOH.

In another embodiment of the present invention, the impregnation of the siliceous-based support is performed by a vapor-phase supporting method using titanium tetrachloride as impregnating agent. Preferably, impregnation is carried out by vapor phase supporting method, wherein the vapor stream is provided by flowing a gas over liquid titanium tetrachloride. The vaporization is conducted at temperatures ranging from 100-400 °C, preferably, from 150-300 °C. Preferably, the gas is an inert gas such as nitrogen, helium, argon, carbon dioxide, and the like. The vapor stream of titanium tetrachloride is then passed over the used catalyst to complete the impregnation step.

The amount of titanium in the siliceous-based support after the impregnation step, will normally be in the range of from 0.1-5 % by weight, preferably 1-4% wt., being particularly preferred 1.5 - 3.0 % wt. based on the total weight of the catalyst.

Following impregnation, the impregnated solids are calcined at a temperature ranging from 450°C to 1000 °C. According to a preferred embodiment of the present invention, the calcination temperature is maintained from 480-900 °C, more preferably from 500-850 °C, being particularly preferred from 520-800 °C, and even more preferred from 550-650 °C. Calcination is preferably performed in the presence of an inert gas which is substantially free of oxygen such as nitrogen, argon, neon, helium or the like or mixture thereof.

In accordance with a preferred embodiment, the calcining step is performed applying at least one temperature ramp of from 100-1000 °C/h, preferably 200-900 °C/h, more preferably from 220-850 °C/h. It has been found that in accordance with a particularly preferred embodiment, the calcining step is performed applying at least one temperature ramp of is from 280-800 °C/h, being particularly preferred from 300-600 °C/h; and most preferred from 360-400 °C/h.

Calcination times are dependent of the calcination temperature reached after the accelerated ramp. Surprisingly it has been found that contrary to what it is mentioned in the prior art , not only temperature but calcination time is a very sensitive variable, and selectivity decreases notably in a very narrow range.Therefore, it has been found that the appropiate selection of temperature ramp, calcination temperature (reached after that ramp) and calcination time results in a catalyst with suitable activity and selectivity. Thus, a particular embodiment of the present invention comprises calcining steps performed by raising the temperature up to 450-1000 °C applying at least one temperature ramp of between 100-1000 °C/h, and afterwards the temperature is maintained from 2-24 hours in order to calcinate the impregnated carrier. In accordance with another embodiment, calcining step is performed by raising the temperature up to 500-850 °C, by applying at least one temperature ramp of between 200-900 °C/h, and maintaining the calcining temperature after each ramp from 2.5-18 hours.According to another preferred embodiment, calcining step is performed by raising the temperature up to 520-800 °C, by applying at least one temperature ramp of between 280-800 °C/h, and maintaining the calcining temperature after each ramp from 2.5-12 hours. According to a preferred embodiment, calcining step is performed by raising the temperature up to 550-650 °C applying at least one temperature ramp of between 300-600 °C/h, and maintaining the calcining temperature after each ramp from 3-12 hours. In a particularly preferred embodiment calcining step is performed by raising the temperature up to 550-650 °C applying a temperature ramp of between 360-400 °C/h and maintaining the calcining temperature from 3-4 hours.

Surprisingly, it has been found also that temperature increase rate does not affect the mechanical properties of the support, and seems to have no influence on the behavior of the final catalyst.

The skilled person in the art would combine the accelerated heating and cooling ramps,and calcining temperature and calcining time features above mentioned in order to optimize the operation procedure without affecting the mechanical properties of the resulting catalyst nor the activity and selectivity in the epoxidation process.

Contrary to what it is generally accepted in the art, the inventors have found that it is possible to apply accelerated heating and, and optionally, cooling rates (heating and cooling ramps), without damaging the physical properties of the siliceous-based support (c.f. results shown in example 2). That constatation, allows to carry out the calcination step in a continuous dryer, preferably in a spray dryer or a rotatory dryer (rotary kiln) or similar.

Optionally, calcination step is performed by applying two, three or more accelerated temperature ramps as those described above, until reaching the temperature of calcination.

After the calcination step, in the vapor phase impregnation method, it is necessary to remove chlorides bonded to titanium and chlorides in excess. For this pourpuse, an hydrolysis step is carried out, feeding water in a hot stream of a gas carrier, usually nitrogen, in the range of from 110 °C to 400 °C preferably from 120 °C to 250 °C,. Afterwards, catalyst is dried for 4-24 hours in the range of from 100 °C to 300 °C preferably from 150 °C to 220 °C, being particularly preferred form 180 to 200 °C, using a stream of gas or air.

It is well known in the art that after calcining, the catalyst may also be treated with an organic silylating agent at elevated temperature. Epoxide selectivity is generally improved by silylation.

Silylation can be performed after calcination, and preceeded by the hydrolysis step in gas phase impregnation.

Nevertheless, it has been demonstrated that, contrary to what it is mentioned in the prior art, it is not neccesary to carry out an hydrolysis step prior to the silylation (c.f. results shown in example 4).

Accordingly, in an embodiment of the present invention the process further comprises:
iii) optionally hydrolysing of the calcinated carrier obtained in step ii); and
iv) silylation of the hydrolysed catalyst obtained in step iii) or alternatively directly silylation of the calcinated carrier obtained in step ii).

In a preferred embodiment, the process is characterized in that no hydrolysis step iii) is performed between calcination step ii) and silylation step iv).

Suitable silylating agents include organosilanes, organohalosilanes, and organodisilazanes. Organosilanes containing from one to three organic substituents may be utilized, including, for example, chlorotrimethylsilane, dichlorodimethyl silane, nitrotrimethyl-silane, chlorotriethylsilane, chlorodimethylphenylsilane and the like. Preferred organohalosilane silylating agents include tetra-substituted silanes having from 1 to 3 halo substituents selected from chlorine, bromine, and iodine with the remainder of the substituents being methyl, ethyl, phenyl or a combination thereof.

Organodisilazanes are represented by the formula R₃Si--NH--SiR₃, wherein the R groups are independently hydrocarbyl groups (preferably, C₁-C₄ alkyl) or hydrogen. Especially preferred for use are the hexaalkyl substituted disilazanes such as, for example, hexamethyldisilazane.

Treatment with the silylating agent may be performed either in the liquid phase (i.e., where the silylating agent is applied to the catalyst as a liquid, either by itself or as a solution in a suitable solvent such as a hydrocarbon) or in the vapor phase (i.e., where the silylating agent is contacted with the catalyst in the form of a gas).

In the event that an hydrolysis step (iii) is performed after the calcination step (ii), silylation may be preceded by a separate drying step, at a temperature ranging from 100 °C to 300 °C preferably from 150 °C to 220 °C, being particularly preferred form 180 to 200 °C.

Therefore, in accordance with a particularly preferred embodiment of the present invention, the process for the preparation of the catalyst comprises:
i) an acid pretreatment of the siliceous-based support;
ii) optionally, directly drying the acid-treated support obtained in step i) at a temperature ranging from 100-300 °C;
iii) directly contacting the acid-treated support obtained in step i) with a titanium-containing agent to obtain a titaniun impregnated support; or alternatively, directly contacting the dried support obtained in step ii) with a titanium-containing agent to obtain a titaniun impregnated support;
iv) calcining the titanium impregnated support obtained in step iii) to obtain the titanium catalyst;
v) directly silylation of the calcinated catalyst obtained in step iv); wherein after step iii) the temperature of the impregnated carrier is rised up to a temperature of from 4500-1000 °C, by applying at least one temperature ramp of between 100-400 °C/h, and maintaining the temperature reached 2 to 24 hours in order to calcinate the impregnated carrier of step iii). In this particular embodiment, no aqueous or alcoholic washing or hydrolysis is performed after the acid treatment step i) nor before the silylation step v).

The heterogeneous catalyst thus obtained is used in oxidation reactions, being particularly useful for catalyzing the epoxidation of olefins with an organic hydroperoxide. Olefin epoxidation processes are well known in the art.

Suitable olefins useful in epoxidation reactions include any olefin having at least one carbon-carbon double bond, and generally from 2 to 30 carbon atoms. Preferably the olefin is an acyclic alkene of from 2 to 10 carbon atoms such as ethylene, propylene, butene, pentene, hexene, heptene, octene, nonene, decene, and isomers thereof. Also preferred are olefinically unsaturated compounds substituted with a hydroxyl group or a halogen group such as allyl chloride or allyl alcohol. A particularly preferred olefin is propylene.

The organic hydroperoxide may be obtained from an hydrocarbon containing at least one secondary or tertiary carbon atom, preferably having from 3 to 20 carbon atoms, more preferably from 3 to 12 carbon atoms. The organic hydroperoxides particularly preferred are secondary alkyl hydroperoxides wherein the hydroperoxy group is on a carbon atom attached directly to an aromatic ring. Accordingly, preferred hydrocarbons include isobutane, ethylbenzene, cyclohexane, isopentane, 2-methylpentane, methylcyclohexane, tetrahydronaphthalene, cumene, diethylbenzene, 3-methylpentane, and the like.

Suitable organic hydroperoxides thus include tertiary butyl hydroperoxide, tertiary amyl hydroperoxide, tertiary hexyl hydroperoxide, tertiary octyl hydroperoxide, ethyl benzene hydroperoxide, cumene hydroperoxide, cyclohexyl hydroperoxide, methyl cyclohexyl hydroperoxide, alpha-ethyl benzyl hydroperoxide, and diisopropylene benzene hydroperoxide. Being particularly preferred the use of ethylbenzene hydroperoxide.

In a particularly preferred embodiment, the catalyst obtained in accordance with the method of the present invention is used in the co-production of propylene oxide and styrene starting from ethylbenzene. In general such process involves the steps of reacting ethylbenzene (EB) with oxygen or air to form ethylbenzene hydroperoxide (EBHP); reacting the ethylbenzene hydroperoxide (EBHP) thus obtained with propene in the presence of an epoxidation catalyst to yield propylene oxide (PO) and 1-phenyl ethanol (methyl phenyl carbinol, MPC); and dehydrating the 1-phenyl ethanol (MPC) into styrene (SM) using a suitable dehydration catalyst.

In the epoxidation reaction, the molar ratio of olefin to hydroperoxide can vary over a wide range and a molar excess of either the olefin or hydroperoxide of up to as high as 100:1 can be used. Preferably, molar ratios of olefin to hydroperoxide varying from about 50:1 to about 1:10 are satisfactory, although it is particularly preferred to employ molar ratios of olefin to hydroperoxide of about 20:1 to about 1:1.

The organic hydroperoxide is prepared by direct oxidation methods, such methods being well-known in the art. For example, molecular oxygen may be passed through the hydrocarbon to convert a portion of the hydrocarbon to the corresponding organic hydroperoxide. Either pure oxygen, air, or oxygen combined with an inert gas such as nitrogen can be used.

The epoxidation reaction may be conducted in the liquid-phase in solvents or diluents that are liquid at the reaction temperature and pressure and are substantially inert to the reactants and the products produced therefrom. Generally, it may be used as a solvent the same hydrocarbon used to produce the organic hydroperoxide reactant. For example, when ethylbenzene hydroperoxide is utilized, the use of ethylbenzene as the epoxidation solvent is preferred.

Preferebly, the ethylbenzene hydroperoxide is pretreated prior to epoxidation reaction. This pretreatment includes at least a basic washing and distillation steps.

The epoxidation reaction may be conducted in the liquid-phase in solvents or diluents that are liquid at the reaction temperature and pressure and are substantially inert to the reactants and the products produced therefrom. Generally, it may be used as a solvent the same hydrocarbon used to produce the organic hydroperoxide reactant. For example, when ethylbenzene hydroperoxide is utilized, the use of ethylbenzene as the epoxidation solvent is preferred.

Suitable reaction temperatures vary from room temperature to 200 °C, preferably from room temperature to 160 °C, more preferably from 70 °C to 120 °C. The reaction is preferably conducted at or above atmospheric pressure. Typical pressures vary from 1 atmosphere to 100 atmospheres, preferably from 30 atmosphere to 70 atmospheres.

The epoxidation may be performed using any of the conventional reactor configurations known in the art for reacting olefin and organic hydroperoxide in the presence of an insoluble catalyst. Continuous as well as batch procedures may be used.

One preferred disposal for continous fixed bed process is a serie of reactors loaded with catalyst. The EBHP solution can be split into diferrent reactors.

In the context of the present invention,, the terms "directly contacting" or "directly drying" or similars, denotes that between the previous step and the referenced step (contacting, drying, ...) it is not performed any intermediate step.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

### Example 1. Acid treatment of the support.

In this example it is demonstrated that contrary to what it is assumed in the prior art, activity and selectivity of the catalyst is good enough when an acid pretreatment of the silicium-based support is performed, but no aqueous or alcoholic washing step is carried out after that acid treatment and before the impregnation step.

There were tested three catalyst:
- Catalyst 1.1 with complete acid pretreatment (including washing).
- Catalyst 1.2 with acid pretreatment but without subsequent washing.
- Catalyst 1.3 without acid pretreatment.

### 1.1. Support with complete pretreatment

### Step A: Acid treatment.

120 g Amorphous pyrogenic silica (Aerolyst®) was charged in a 500 mL round-bottom flask and 360 ml of 4N hydrochloric acid was added. The mix was heated to 70°C and maintained at this temperature for 4 hours without stirring. The convection of the hot and cold parts of the liquid generates a fluid motion that keeps it smooth. The acid ratio was 120 grams support/360 ml of acid. After 4 hours of treatment, flask was cooled slightly.

### Step B: Washing

The content was transferred to a packed column of 400 ml capacity with filter plate on the bottom, which will retain the solid. The liquid was returned to the flask successively to drag the particles adhered to the walls.

Once the support was in the packed column, this was coupled to an impeller pump to wash the carrier with demineralized water.

To control washing evolution, a conductivity cell was used, which gives a continuous reading of the electrical conductivity of the washing liquid. Initial conductivity is 377 mS/cm. This electrical conductivity is influenced mainly by the CI-anion, so that when this stop lowering may assume that the washing process has been completed. Final conductivity was 0,0023 mS/cm.

| H₂O Volume (ml) | Conductivity (mS/cm) |
|---|---|
| 0 | 377 |
| 100 | 520 |
| 200 | 419 |
| 300 | 320 |
| 400 | 248 |
| 500 | 189,8 |
| 600 | 146,2 |
| 700 | 112,6 |
| 800 | 87,7 |
| 900 | 65 |
| 1000 | 34,8 |
| 1100 | 26,3 |
| 1200 | 21,7 |
| 1300 | 13,88 |
| 1400 | 6,7 |
| 1500 | 2,17 |
| 1600 | 0,494 |
| 1700 | 0,1098 |
| 1800 | 0,0281 |
| 1900 | 0,00779 |
| 2000 | 0,00378 |
| 2100 | 0,00296 |
| 2200 | 0,00253 |
| 2300 | 0,00229 |

Washing flow was about 4 ml/minute, with a conductivity reading every 100 ml.

Total elution volumes was 2.3 liters of demineralized water. This volume includes the amount of fluid that remains in the column at the time of stopping the wash.

Once the support was washed, a stream on nitrogen was passed through the solid, and then the column was drained by gravity. In order to eliminate water prior to impregnation, an oven was used at 110°C for 15 h.

### Step C: Impregnation

30 g of treated support with carborundum (SiC) was then placed in a quartz reactor for vapor-phase impregnation. Temperature raising rate was 100°C/h. A nitrogen stream of 60 NL/h was passed through the bed for 4 hours at 200°C. Then 3,4 g of titanium tetrachloride was feed in 90 minutes at 200°C, using the stream of nitrogen as carrier. The nitrogen stream was maintained 1 hour more.

### Step D: Calcination

The support was then heated to a temperature between 550-650°C °C at 100°C/h rate and calcined at this temperature for 7 hours. Then it was allow cooling to 200 °C.

### Step E: Hydrolysis

Hydrolysis was carried out at 200 g feeding 40 cm³/h of water in 60 NL/h of nitrogen for 2 hours. Then support was dried for 12 hours at 200°C using a stream of 30 NL/h of nitrogen.

### Step F: Silylation

A 500mL round-bottom flask was equipped with a condenser and a thermometer. The flask was charged with 165 cm³ of toluene, 12,5 g of hexamethyldisilazane and 25 g of catalyst. The system was heated to reflux (110°C) and kept refluxing for 3 hours. The system was cooled down and the catalysis was filtered and dried with a nitrogen stream. Them the catalyst was completely dried in an oven at 120 °C.

### 1.2. Support with acid pretreatment but without washing

One proceeds as described above, but after step A (4 hours of acid treatment) the solid was filtered and dried in an oven at 110 °C for at least 4 hours to eliminate water. Support was then placed in a reactor for steps C +D + E +F.

### 1.3. Support without acid pretreatment

No A, neither B steps. Support was placed in a reactor for steps C +D + E +F.

### Evaluation of the performance of the catalyst

To evaluate the performance of the catalysts, batch epoxidations of propylene with ethylbenzene hydroperoxide were carried out. The procedure is described as follows:
The catalyst performance was tested in a batch stirred tank reactor. The reactor volume was 1 liter and the catalyst was put on a basket inside the reactor. The catalyst loading was 3g.

The epoxidation of propene was carried out with a solution of ethylbenzene hydroperoxide. The ethylbenzene hydroperoxide contains 35% wt. of hydroperoxide in ethylbenzene and it was made by oxidation of ethylbenzene with air. 150 g of ethylbenzene hydroperoxide solution and 250 g of propylene were introduced in the reactor. The reactor was heated at 100°C. Once the reactor reached the set temperature (100°C) several samples (0, 90, 150 and 300 min) were taken from the reactor in order to analyze the composition by GC and HPLC. The total epoxidation time was 300 min.

Batch epoxidation tests were repeated three days with fresh feed and same catalyst in order to check the lost in activity/selectivity.

Table 1 shows the results obtained regarding activity (conversion of EBHP) and molar selectivity to ACP..

As well as activity, selectivity is a critical parameter, as the percentage of ACP are process losses. Each percentage point ACP does not occur, consolidated, represent an important advance. A typical value in certain epoxidation technologies may be 4% and any technology below 2% is to reduce losses by this concept by half. Back to spend 2% to 1% is a success.

**Table 1.**

| | 1.1 | 1.2 | 1.3 |
|---|---|---|---|
| | Acid pretreatment | Acid pretreatment (no washing) | No pretreatment |
| **Reaction 1** | | | |
| k' (L/hg) | 0,20 | 0,21 | 0,18 |
| Activity (%) | 100% | 105% | 86% |
| S ACP/EBHP | 2,1% | 1,4% | 1,4% |

| **Reaction 2** | | | |
|---|---|---|---|
| k' (L/hg) | 0,17 | 0,16 | 0,13 |
| Activity (%) | 100% | 99% | 79% |
| S ACP/EBHP | 1,5% | 0,7% | 1,3% |

| **Reaction 3** | | | |
|---|---|---|---|
| k' (L/hg) | 0,15 | 0,15 | 0,11 |
| Activity (%) | 100% | 98% | 79% |
| S ACP/EBHP | 1,4% | 1,0% | 0,6% |

| | | | |
|---|---|---|---|
| Notes: 1. **k':** pseudo-kinetic rate constant, considering propylene concentration constant during epoxidation reaction 2. **Activity:** ratio between k' of a sent or regenerated catalyst and k' of fresh catalyst 3. **S ACP/EBHP:** molar selectivity of ACP | | | |

As shown in the table above, with acid treatment and no washing (example 1.2):
- We save a step (water and energy saving)
- Activity is as good as with complete pretreatment (example 1.1)
- Selectivity is as good or better than complete pretreatment

### Example 2:

Influence of heating and cooling ramps on the mechanical properties of the support, using the same supplier siliceous support in every case:
Sample 0: Supplier siliceous support (Aerolyst®).
Sample 1: The support (sample 0) was heated in an oven to 200 °C in 30 min (360 °C/h), staying an hour at 200°C in the inner of the oven and directly sample extraction at room temperature.
Sample 2. The support (sample 0) was heated in an oven to 200 °C for 30 min (360 °C/h), staying an hour at 200°C in the inner of the oven, continued heating from 200 to 600 °C for 30 min (800 °C/h), staying an hour at 600°C in the inner of the oven and removal of the sample directly to room temperature.
Sample 3. The support (sample 0) was heated in an oven to 200 °C for 30 min (360 °C /h), staying an hour at 200°C in the inner of the oven, continued heating from 200 to 600 °C for 30 min (800 °C/h), staying an hour at 600°C in the inner of the oven, continued heating from 600 °C to 800 °C in 30 min (400 °C/h), staying an hour at 800°C in the inner of the oven and removal of the sample directly to room temperature.

Then physical properties of supports were compared, using the physisorption isotherm of an inert gas (nitrogene) ASTM D3663-03 (2008) and ASTM: D 4284 - 83

Table 2 shows the results obtained, wherein it is observed that the mechanical properties of the catalyst remain unchanged:

**Table 2.**

| T^{a} (atm N₂) | Surface(m²/g) | SPV (cc/g) | PD (N₂) Å |
|---|---|---|---|
| Room Temp. | 161 | 0,94 | 175 |
| 200 | 162 | 0,96 | 174 |
| 600 | 161 | 0,96 | 174 |
| 800 | 160 | 0,96 | 174 |

| | | | |
|---|---|---|---|
| Surface: BET surface area SPV: Specific Pore Volume PD: Average Pore Diameter | | | |

### Example 3:

Manufacture of catalyst calcination over at diferrent calcination times.

Following the preparation process as described above in example 1, the following catalysts are prepared:
3.1. Calcination above 550 °C during t = 2 h
3.2. Calcination above 550 °C during t = 2,5 h
3.3. Calcination above 550 °C during t = 3 h
3.4. Calcination above 550 °C during t = 7 h
Catalyst 3.1: Steps A+C+E+F as described in example 1. In calcination step (D) temperature is risen by 380°C/h an the calcination time over 550°C is 2 hours.
Catalyst 3.2: Steps A+C+E+F as described in example 1, In calcination step (D) temperature is risen by 380°C/h an the calcination time over 550°C is 2,5 hours.
Catalyst 3.3: Steps A+B+C+E+F as described in example 1. In calcination step (D) temperature is risen by 100°C/h an the calcination time over 550°C is 3 hours.
Catalyst 3.4: Steps A+B+C+E+F as described in example 1. In calcination step (D) temperature is risen by 100°C/h an the calcination time over 550°C is 7 hours.

To evaluate the performance of the catalysts, batch epoxidations of propylene with ethylbenzene hydroperoxide were carried out. The procedure is described in Example 1.

Table 3 shows the results obtained. As a result of the process according to the present invention, it is not necessary long calcination times, so energy is saved and catalyst has same behavior in conversion, but there is a minimum value below which the selectivity decreases rapidly.:

**Table 3.**

| | **Example 3.1** | **Example 3.2** | **Example 3.3** | **Example 3.4** |
|---|---|---|---|---|
| t>550°C (h) | 2 | 2,5 | 3 | 7 |

| **Reaction 1** | | | | |
|---|---|---|---|---|
| k' (L/hg) | 0,11 | 0,16 | 0,18 | 0,19 |
| Activity (%) | 57% | 82% | 95% | 100% |
| S ACP/EBHP | 0,46% | 1,52% | 0,98% | 1,35% |

| **Reaction 2** | | | | |
|---|---|---|---|---|
| k' (L/hg) | 0,11 | 0,13 | 0,15 | 0,14 |
| Activity (%) | 79% | 88% | 107% | 100% |
| S ACP/EBHP | 0,50% | 1,51% | 0,73% | 1,11% |

| **Reaction 3** | | | | |
|---|---|---|---|---|
| k' (L/hg) | 0,11 | 0,12 | 0,14 | 0,14 |
| Activity (%) | 76% | 87% | 99% | 100% |
| S ACP/EBHP | 0,97% | 1,24% | 0,86% | 1,50% |

| | | | | |
|---|---|---|---|---|
| Notes: 1. **k':** pseudo-kinetic rate constant, considering propylene concentration constant during epoxidation reaction 2. **Activity:** ratio between k' of a sent or regenerated catalyst and k' of fresh catalyst 3. **S ACP/EBHP:** molar selectivity of ACP | | | | |

### Example 4: Effect of hidrolysis step.

Two catalysts were prepared following general procedure as described in example 1:
Catalyst 4.1 with hydrolysis after gas-phase impregnation and prior silylation: Steps A+B+C+D+E+F
Catalyst 4.2 without hydrolysis prior silylation. Steps A+B+C+D+F

To evaluate the performance of the catalysts, batch epoxidations of propylene with ethylbenzene hydroperoxide were carried out. The procedure is described in example 1.

Table 4 shows the results obtained, wherein it is observed the same behavior in conversion:

**Table 4:**

| | 4.1 Hydrolisis | 4.2 No Hydrolisis |
|---|---|---|
| **Reaction 1** | | |
| k' (L/hg) | 0,19 | 0,18 |
| Activity (%) | 100% | 95% |
| S ACP/EBHP | 1,4% | 2,4% |

| **Reaction 2** | | |
|---|---|---|
| k' (L/hg) | 0,14 | 0,14 |
| Activity (%) | 100% | 101% |
| S ACP/EBHP | 1,1% | 2,8% |

| Reaction 3 | | |
|---|---|---|
| k' (L/hg) | 0,14 | 0,15 |
| Activity (%) | 100% | 102% |
| S ACP/EBHP | 1,5% | 1,2% |

| | | |
|---|---|---|
| Notes: 1. **k':** pseudo-kinetic rate constant, considering propylene concentration constant during epoxidation reaction 2. **Activity:** ratio between k' of a sent or regenerated catalyst and k' of fresh catalyst 3. **S ACP/EBHP:** molar selectivity of ACP | | |

### Example 5: Silylation

Two catalysts were prepared following the general procedure as described in example 1:
Catalyst 5.1 with silylation after impregnation. Steps A+B+C+D+E+F
Catalyst 5.2 without silylation after impregantion. Steps A+B+C+D+E

The catalyst performance was tested in a batch stirred tank reactor. The reactor volume was 1 liter and the catalyst is put on a basket inside the reactor. The catalyst loading was 4g.

The epoxidation of propene was carried out with a solution of ethylbenzene hydroperoxide. The ethylbenzene hydroperoxide contains 35%w of hydroperoxide in ethylbenzene and it was made by oxidation of ethylbenzene with air. 150 g of ethylbenzene hydroperoxide solution and 250 g of propylene were introduced in the reactor. The reactor was heated at 100°C. Once the reactor reached the set temperature (100°C) several samples (0, 90, 150 and 300 min) were taken from the reactor in order to analyze the composition by GC and HPLC. The total epoxidation time was 300 min.

Each reaction is repeated three days with fresh feed and same catalyst, in order to check the lost in activity/selectivity.

Table 5 shows the results obtained, wherein it is observed that silylating catalysis has a clear improvement in activity and selectivity.

| | Sylilating | No Sylilating |
|---|---|---|
| **Reaction 1** | | |
| k' (L/hg) | 0,18 | 0,14 |
| Activity (%) | 100% | 81% |
| S ACP/EBHP | 1,2% | 3,9% |

| **Reaction 2** | | |
|---|---|---|
| k' (L/hg) | 0,15 | 0,09 |
| Activity (%) | 100% | 62% |
| S ACP/EBHP | 0,9% | 2,0% |

| **Reaction 3** | | |
|---|---|---|
| k' (L/hg) | 0,12 | 0,08 |
| Activity (%) | 100% | 73% |
| S ACP/EBHP | 1,4% | 2,9% |

| | | |
|---|---|---|
| Notes: 1. **k':** pseudo-kinetic rate constant, considering propylene concentration constant during epoxidation reaction 2. **Activity:** ratio between k' of a sent or regenerated catalyst and k' of fresh catalyst 3. **S ACP/EBHP:** molar selectivity of ACP | | |

### REFERENCES CITED IN THE APPLICATION

1. US5932751
2. "Halide Effects in Transition Metal Catalysis" Keith Fagnou and Mark Lautens. Angewandte. Chemie. Int. Ed. 2002, 41, 26 - 47
3. "Mecanism in Homogeneus and heterogeneus epoxidation catalyst" S.Ted Oyama. Ed Elsevier, Pag 251-252
4. "Fundamentos de ciencia de los materiales" Tomo I. Vicente Amigó Borrás; Carlos Ferrer Giménez; María Dolores Salvador Moya; Francisco Segovia López; Alfonso Cristóbal Cárcel González; Universidad Politécnica de Valencia Departamento de Ingeniería Mecánica y de Materiales Valencia : Universidad Politécnica de Valencia 1994, Chapter 11 Características térmicas de los materiales, apartado 4.5: Efecto de los gradientes de temperatura: roturas por choque térmico. (page 405)
5. EP345856
6. EP1292391
7. ASTM D3663 - 03(2008)
8. ASTM:4641-12
9. Barrett, E.P.; L.G. Joyner, P.P. Halenda (1951). "The Determination of Pore Volume and Area Distributions in Porous Substances. I. Computations form Nitrogen Isotherms". J. Am. Chem. Soc. 73
10. ASTM: D 4284 - 83

## Claims

1. A process for the preparation of a titanium-based catalyst comprising:
i) contacting a siliceous-based carrier with a titanium-containing agent to obtain an impregnated carrier;
ii) raising the temperature up to 450-1000 °C, applying at least one temperature ramp of between 100-1000 °C/h, and maintaining the temperature reached after each ramp from 2 to 24 hours in order to calcinate the impregnated carrier of step i).

2. The process according to claim 1, wherein the temperature is raised up to 500-850 °C, the temperature ramp are of between 200-900 °C/h, and the time of maintenance of the temperature after each ramp ranges from 2.5 to 18 hours

3. The process according to any of the claims 1-2, wherein the temperature is raised up to 550-650 °C, the temperature ramp are of between 300-600 °C/h, and the time of maintenance of the temperature after each ramp ranges from 3-12 hours.

4. The process according to any of the claims 1-3, wherein the process further comprises an acid treatment to the siliceous-based carrier previously to step i), the acid treatment being carried out with an aqueous solution of a strong acid selected from sulfuric acid, hydrochloric acid, phosphoric acid, nitric acid, and acetic acid.

5. The process according to claim 4, wherein the acid treatment comprises contacting the siliceous-based carrier with an aqueous solution of hydrochloric acid at a temperature ranged from 20 to 90 °C; for a period of from 1-24 h.

6. The process according to any of the claims 4-5, wherein the impregnation step is carried out directly after the acid treatment, or alternatively a drying step is carried out directly on the acid treated carrier followed by the impregnation step i).

7. The process according to any of the claims 1-6 which further comprises:
iii) optionally hydrolysing the calcinated carrier obtained in step ii); and
iv) silylation of the hydrolysed catalyst obtained in step iii) or alternatively, of the calcinated carrier obtained in step ii)

8. The process according to claim 7, wherein the silylation step iv) is carried out directly after calcination step ii).

9. The process according to any of claims 7-8, wherein silylation is carried out with a silylating agent selected from organosilanes, organohalosilanes, and organodisilazanes, and mixtures thereof; preferably the silylating agent is hexamethyldisilazane.

10. The process according to any of the claims 1-9, wherein the titanium-containing agent is selected from the group consisting of:
a) a solution of a titanium alkoxide or a titanium halide;
b) a vapor stream of a titanium halide.

11. The process according to claim 10, wherein the titanium halide is titanium tetrachloride.

12. The process according to any of the claims 1-11, wherein the siliceous-based carrier is an amorphous pyrogenic or amorphous precipitated silica.

13. The process according to any of the claims 1-12, wherein the calcination step is carried out in a continuous dryer.

14. A process for the preparation of an alkylene oxide comprising:
a) carrying out the process of preparation of a heterogeneous titanium-based catalyst of any of the claims 1-13; and
b) reacting an olefin with an organic hydroperoxide in the presence of the heterogeneous titanium-based catalyst of step a).

15. The process according to claim 14, wherein the alkylene oxide is propylene oxide and its preparation process comprises reacting ethylbenzene hydroperoxide with propene in the presence of the heterogeneous catalyst to yield propylene oxide and 1-phenyl ethanol.
